(19) Europäisches Patentamt
European Patent Office
Office européen des brevets

(11) EP 1 498 131 B1

(12) # EUROPEAN PATENT SPECIFICATION

(45) Date of publication and mention of the grant of the patent:
**19.03.2014 Bulletin 2014/12**

(51) Int Cl.:
***A61K 36/64*** (2006.01) ***A61P 25/28*** (2006.01)

(21) Application number: **03016069.1**

(22) Date of filing: **15.07.2003**

(54) **Medicinal preparation containing phenylethanoid glycosides extracted from Cistanche tubulosa**

Phenylethanoidglykosidextrakte aus Cistanche Tubulosa als Arzneimittel

Préparation pharmaceutique contenant des glycosides phényléthanoides extraites de Cistanche tubulosa

(84) Designated Contracting States:
**AT BE BG CH CY CZ DE DK EE ES FI FR GB GR HU IE IT LI LU MC NL PT RO SE SI SK TR**

(43) Date of publication of application:
**19.01.2005 Bulletin 2005/03**

(73) Proprietor: **Sinphar Pharmaceutical Co., Ltd.**
**Taipei (TW)**

(72) Inventors:
- **Tu, Pengfei**
  **Peking,**
  **China 100083 (CN)**
- **Song, Zhihong**
  **Peking,**
  **China 100083 (CN)**
- **Lei, Li**
  **Peking,**
  **China 100083 (CN)**

(74) Representative: **Grünecker, Kinkeldey, Stockmair & Schwanhäusser**
**Leopoldstrasse 4**
**80802 München (DE)**

(56) References cited:
- **KOBAYASHI H ET AL: "NEW PHENYLETHANOID GLYCOSIDES FROM CISTANCHE-TUBULOSA SCHRENK HOOK. F. I" CHEMICAL AND PHARMACEUTICAL BULLETIN (TOKYO), vol. 35, no. 8, 1987, pages 3309-3314, XP001161005 ISSN: 0009-2363**
- **YOSHIZAWA F ET AL: "CONSTITUENTS OF CISTANCHE TUBULOSA (SCHRENK) HOOK. F. II. ISOLATION AND STRUCTURES OF A NEW PHENYLETHANOID GLYCOSIDE AND A NEW NEOLIGNAN GLYCOSIDE" CHEMICAL AND PHARMACEUTICAL BULLETIN, PHARMACEUTICAL SOCIETY OF JAPAN. TOKYO, JP, vol. 38, no. 7, July 1990 (1990-07), pages 1927-30, XP002140954 ISSN: 0009-2363**
- **AHMAD M ET AL: "ACTEOSIDE: A NEW ANTIHYPERTENSIVE DRUG" PHYTOTHERAPY RESEARCH, JOHN WILEY & SONS LTD. CHICHESTER, GB, vol. 9, no. 7, 1995, pages 525-527, XP008007439 ISSN: 0951-418X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## FIELD OF THE INVENTION

The present invention relates generally to a medicinal preparation derived from a herbaceous plant, and more particularly to a medicinal preparation containing phenylethanoid glycosides which are extracted from a herbaceous plant belonging to the genus *Cistanche.* The preparation is used as an active ingredient of the drug capable of prevention of senile dementia. The present invention also covers a process for making the medicinal preparation as well as the uses of the medicinal preparation.

## BACKGROUND OF THE INVENTION

The senior citizens are generally vulnerable to various physical and mental impairments for which there is often no panacean remedy. The senile dementia is a case in point. However, it is clinically evident that the fleshy stems of herbs belonging to the genus *Cistanche* are effective in treatment of infertility, impotency, constipation, etc. In addition, the preparation made from the fleshy stems of such perennial herbs as described above is nourishing to blood and kidney. These parasitic and perennial herbs are widely cultivated in the northwestern provinces of China and are locally known as "desert ginseng". The most abundantly cultivated species of the genus *Cistanche* is *Cistanche tubulosa (Schrenk.) Wight.*

According to the systematic research done by the Japanese scientists on the chemical constituents and the pharmacological activities of the perennial herbs (genus *Cistanche*), phenylethanoid glycosides are the principal active ingredients of these perennial herbs. Such active ingredients are effective antioxidants, metabolic promoters, memory enhancers, sexuality enhancers, etc. The medicinal properties of various phenylethanoid glycoside compounds have been studied by many researchers. For such information, please refer to the following publications: Sato T., et al. Yakugaku Zasshi, 1985, 105 (12): 1131; Jimenez C., et al. Nat Prod Rep, 1994, 11 (6): 591; Cometa F., et al. Fitoterapia, 1993, 64 (3): 195.

## SUMMARY OF THE INVENTION

The inventors of the present invention have had more than ten years of research experiences on the perennial herbs of the genus *Cistanche.* Among all species of the genus *Cistanche,* one species, *Cistanche tubulosa (Schrenk.) Wight*, contains the greatest amount of phenylethanoid glycoside compounds. The inventors of the present invention introduce a novel process for extracting the phenylethanoid glycosides from *Cistanche tubulosa (Schrenk.) Wight*, as well as a medicinal preparation containing phenylethanoid glycosides. As a result of a number of pharmacological tests conducted by the inventors of the present invention, the medicinal preparation was found to have a significant effect on memory enhancement, thrombosis prevention, inhibition of blood platelets aggregation, etc.

The primary objective of the present invention is to provide a medicinal preparation containing phenylethanoid glycosides which are extracted from a herbaceous plant, *Cistanche tubulosa (Schrenk.) Wight*. It is another objective of the present invention to provide a process for making the medicinal preparation containing phenylethanoid glycosides. It is still another objective of the present invention to provide a medicinal composition for prevention of senile dementia.

The medicinal preparation of the present invention contains 25-70% of echinacoside by weight of the preparation, and 1-40% of acteoside by weight of the preparation.

Preferably, the medicinal preparation of the present invention is made from the fleshy stems of *Cistanche tubulosa (Schrenk.) Wight*.

Preferably, the medicinal preparation of the present invention further contains 2'-acetylacteoside; campneoside I; campneoside II; cistantubuloside A, $B_1$, $B_2$, $C_1$, $C_2$; crenatoside; decaffeoylacteoside; isoacteoside; rhodioloside; syringalide A; 3'-$\alpha$-L-rhamnopyranoside, and tubuloside A, with each in an amount less than 5% by weight of the medicinal preparation.

The process according to claim 4 of the present invention for making the medicinal preparation involves a first step in which the subterranean portions of *Cistanche tubulosa (Schrenk.) Wight* are extracted by a first polar solvent. The extract so obtained is then introduced into a column which is packed with hydrophobic macro-porous polymeric beads, thereby enabling phenylethanoid glycosides to be adsorbed on the polymeric beads. The relatively less strongly adsorbed compounds are then eluted from the column by use of a second polar solvent serving as a mobile phase, with most of phenylethanoid glycosides still being adsorbed on the polymeric beads. Finally, phenylethanoid glycosides are eluted from the column by use of a third polar solvent so as to obtain an eluate which contains phenylethanoid glycosides. The third polar solvent is lower in polarity than the second polar solvent.

Preferably, the subterranean portions of *Cistanche tubulosa (Schrenk.) Wight* are fleshy stems. The subterranean portions which are mixed with the first polar solvent, and the resulting mixture is then cooked to boil for a period

lasting 0.5-10 hours. The mixture is subsequently filtered so as to obtain a solution. The solution is an extract, which may be in a concentrated form by decompression. Preferably, the mixture contains the subterranean portions of *Cistanche tubulosa (Schrenk.) Wight* and the first polar solvent in a weight ratio ranging from 1:4 to 1:20. The first polar solvent is either water, or a mixed solvent containing water and ethyl alcohol. Preferably, the polymeric bead of the column used in the process of the present invention is a cross-linked polyaromatics. More preferably, the polymeric bead is formed of cross-linked polystyrene, or a cross-linked copolymer of styrene and divinyl benzene. The second polar solvent is water. The third polar solvent is methanol, ethanol, a water-methanol mixture, or a water-ethanol mixture. More preferably, the third polar solvent is a mixture of water and ethanol.

The process of the present invention further comprises removing the solvent that is contained in the eluate so produced, thereby resulting in production of a dry preparation.

The preparation of the present invention can be used to make a medicinal composition for prevention of senile dementia. The medicinal composition contains a therapeutically effective amount of the preparation as an active ingredient and a pharmaceutically acceptable carrier or diluent for the active ingredient.

The medicinal composition contains a therapeutically effective amount of the preparation and a medicinally-allowable carrier or diluent. The preparation is used as an active ingredient of the compound.

The present invention also discloses a use of the preparation of the present invention in the manufacture of a medicament for preventing and treating senile dementia disease in a patient.

Detailed Description of the Invention

The present invention provides a preparation containing phenylethanoid glycoside compounds from *Cistanche tubulosa (Schrenk.) Wight*, which contains 25-70% of echinacoside by weight of the preparation, and 5-40% of acteoside by weight of the preparation. The phenylethanoid glycoside compounds derived from *Cistanche tubulosa (Schrenk.) Wight* have the following chemical structure:

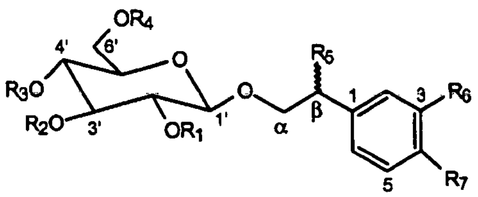

and include 2'-acetylacteoside; campneoside I; campneoside II; cistantubuloside A, $B_1$, $B_2$, $C_1$, $C_2$; crenatoside; decaffeoylacteoside; isoacteoside; rhodioloside; syringalide A; 3'-$\alpha$-L-rhamnopyranoside, and tubuloside A shown in Table 1, in which most of the compounds are in a minute or trace amount in the preparation except echinacoside and acteoside.

Table 1 principal ingredients of the preparation

| Ingredient | $R_1$ | $R_2$ | $R_3$ | $R_4$ | $R_5$ | $R_6$ | $R_7$ |
|---|---|---|---|---|---|---|---|
| 2'-Acetylacteoside | Ac | Rha | Cf | H | H | OH | OH |
| Acteoside | H | Rha | Cf | H | H | OH | OH |
| Campneoside I | H | Rha | Cf | H | OMe(S/R) | OH | OH |
| Campneoside II | H | Rha | Cf | H | OH(S/R) | OH | OH |
| *Cistantubuloside A | H | Rha | Cf | Glc | H | H | OH |
| *Cistantubulosides $B_1$/$B_2$ | H | Rha | Cm/c-Cm | Glc | H | OH | OH |
| *Cistantubulosides $C_1$/$C_2$ | H | Rha | Cf | Glc | OH(S/R) | OH | OH |
| Decaffeoylacteoside | H | Rha | H | H | H | OH | OH |
| Echinacoside | H | Rha | Cf | Glc | H | OH | OH |
| Isoacteoside | H | Rha | H | Cf | H | OH | OH |
| Rhodioloside (Salidroside) | H | H | H | H | H | H | OH |
| Syringalide A | | | | | | | |
| 3'-$\alpha$-L-rhamnopyrano side | H | Rha | Cf | H | H | H | OH |
| Tubuloside A | Ac | Rha | Cf | Glc | H | OH | OH |

(continued)

| Ingredient | $R_1$ | $R_2$ | $R_3$ | $R_4$ | $R_5$ | $R_6$ | $R_7$ |
|---|---|---|---|---|---|---|---|
| Crenatoside | | | | See following structure | | | |
| *new compound<br>Ac: Acetyl Cf: trans-Caffeoyl Cm: trans-Coumaroyl c-Cm: cis-Coumaroyl Glc: β-D-Glucopyranose Rha: α-L-Rhamnopyranose | | | | | | | |

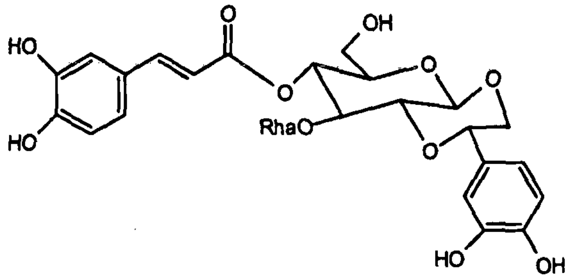

crenatoside

All the compounds listed in Table 1 were verified by means of a high performance liquid chromatography, which was obtained under the following conditions: stationary phase being silicone of C18 alkyl silane; mobile phase being acetonitrile-0.05M phosphoric acid aqueous solution (gradient eluting (4:96→15:85)), flow rate of 1 ml/min, and detecting wavelength of 330nm.

The process of making the medicinal preparation of the present invention is explicitly described hereinafter. The process of the present invention involves two steps, which are extraction and purification. In the first step, the fleshy stems of *Cistanche tubulosa (Schenk.) Wight* are cut into flakes or are comminuted into fine particles or powder. The flakes or fine particles are then soaked in a medium which is water, or a mixture of water and ethanol. The extraction is carried out at room temperature. The filtrate is concentrated in vacuo, thereby resulting in formation of an extract. The purification of the extract is done by heating the extract in water before the extract is transferred to an adsorption column packed with macro-porous adsorption resin of D-101 type or AB-8 type. The column is eluted with water. The eluting may be carried out with a solution of a constant concentration or solutions of gradient concentrations. The eluate is collected, concentrated and then dried by a conventional drying method. Upon completion of the drying of the eluate, the medicinal preparation of the present invention is obtained. The medicinal preparation so produced contains phenylethanoid glycosides and is used for making medicinal compositions for use in prevention of senile dementia.

The pharmacological test of the medicinal preparation of the present invention was carried out by a so-called "water maze experiment", in which a test group of small mice was provided with a diet containing the medicinal preparation of the present invention. In comparison with the control group, the learning memory of the small mice of the test group were enhance, the memory loss of the test group due to ethanol or drugs was apparently reduced. Similarly, a group of rats was provided with a diet containing the medicinal preparation is also disclosed herein. By comparison, these rats were found to be less vulnerable to thrombosis, or aggregation of blood platelets. On the basis of the results of the pharmacological test described above, a clinical test may be carried out to study the effect of the medicinal preparation of the present invention on prevention and treatment of senile dementia, including VD and AD.

The extraction, purification, and pharmacological effect of the medicinal preparation of the present invention will be further explained by referring to the following nonrestrictive embodiments and comparative experiments.

EXTRACTION:

Embodiment 1

10 kg of the flakes of fleshy stems of *Cistanche tubulosa (Schrenk.) Wight* was soaked in water in an amount which was 8 times of the flakes. The flakes was soaked in the water for one hour before being decocted with the water for two hours. The decocted mixture was filtered to obtain a first filtrate. The residue was then decocted with the water in an amount which was 6 times of the residue and the decocted mixture was filtered to obtain a second filtrate. A third filtrate was also obtained by the same procedures as the second filtrate. The three filtrates were combined and concentrated in vacuo to have a specific gravity of 1.10 (50 °C). The filtrate in the concentrated form was mixed with ethanol

to form a mixture containing 60% of the ethanol, which was then refrigerated for 12 hours. Thereafter, a supernatant was harvested from the cooled mixture while the residue was filtered to obtain a filtrate, which was combined with the supernatant to form an end extract. The end extract was concentrated in vacuo to have a specific gravity of 1.10 (50°C), in which the ethanol was recycled. The end extract so produced has a weight of 5.6 kg.

Embodiment 2

10 kg of the fleshy stems of *Cistanche tubulosa (Schrenk.) Wight* in the powder form was soaked in water in an amount which was 15 times of the powder for 2 hours, and was then decocted for 3 hours. Thereafter, the decocted mixture was filtered to obtain a first filtrate, while the residue of the decocted mixture was mixed with water in an amount which was 12 times of the residue, decocted for 2 hours and filtered to obtain a second filtrate. The procedures were repeated two additional times to obtain a third filtrate and a fourth filtrate. The four filtrates were combined and concentrated in vacuo to have a specific gravity of 1.25 (50 °C). The filtrate in the concentrated form was mixed with ethanol to form a mixture containing 80% of the ethanol, which was then refrigerated for 24 hours. Thereafter, a supernatant was harvested from the cooled mixture while the residue was filtered to obtain a filtrate, which was combined with the supernatant to form an end extract. The end extract was concentrated in vacuo to have a specific gravity of 1.25 (50°C), in which the ethanol was recycled. The end extract so produced has a weight of 7.2 kg.

Embodiment 3

10 kg of the flakes of fleshy stems of *Cistanche tubulosa (Schrenk.) Wight* was soaked in water in an amount which was 7 times of the flakes. The flakes was soaked in the water for three hours before being decocted with the water for four hours. The decocted mixture was filtered to obtain a first filtrate. The residue was then decocted with the water in an amount which was 5 times of the residue and the decocted mixture was filtered to obtain a second filtrate. A third filtrate and a fourth filtrate were also obtained by the same procedures as the second filtrate. The four filtrates were combined and concentrated in vacuo to have a specific gravity of 1.05 (50°C). The filtrate in the concentrated form was mixed with ethanol to form a mixture containing 50% of the ethanol, which was then refrigerated for 10 hours. Thereafter, a supernatant was harvested from the cooled mixture while the residue was filtered to obtain a filtrate, which was combined with the supernatant to form an end extract. The end extract was concentrated in vacuo to have a specific gravity of 1.10 (50°C), in which the ethanol was recycled. The end extract so produced has a weight of 6.5 kg.

Embodiment 4

10 kg of the fleshy stems of *Cistanche tubulosa (Schrenk.) Wight* in the powder form was soaked in 40% ethanol in an amount which was four times of the powder for 3 hours, and was then decocted under refluxing for 3 hours. Thereafter, the decocted mixture was filtered to obtain a first filtrate, while the residue of the decocted mixture was mixed with 40% ethanol in an amount which was four times of the residue, decocted for four hours and filtered to obtain a second filtrate. The procedures were repeated two additional times to obtain a third filtrate and a fourth filtrate. The four filtrates were combined and concentrated in vacuo to have a specific gravity of 1.05 (50°C), thereby resulting in production of an end extract having a weight of 6.2 kg.

PURIFICATION:

Embodiment 5

6 kg of the end extract was dissolved in water with heating, which was in an amount of one half of the end extract. The extract solution was then applied into an adsorption column packed with pretreated macro-porous adsorption resin of the D-101 type. The column was first eluted with water to yield a water eluate in the amount of two times of the fleshy stems, and was than eluted with 20% ethanol to yield a first 20% ethanol eluate in the amount of two times of the fleshy stems. The water eluate was subjected to another round of the adsorption-desorption operations to obtain a second ethanol eluate. The two 20% ethanol eluates were combined, concentrated, and dried to yield a preparation containing phenylethanoid glycosides and having a weight of 865 g.

The contents of echinacoside and acteoside were measured by a high performance liquid chromatography (HPLC), with the stationary phase being silicone of C18 alkyl silane, with the mobile phase being methanol-0.15% acetic acid (30:70), with the flow rate of 1 ml/min, and with the detecting wavelength of 333 nm.

The echinacoside and the acteoside dried at 60°C in vacuo for 24 hours were measured and dissolved in 50% methanol to prepare reference solutions, with each 1 ml solution containing 0.1 mg of the solute.

The test solution was prepared by dissolving 50 mg of the preparation containing phenylethanoid glycosides

in an appropriate amount of 50% methanol in a 25 ml-graduated container while sonicating. More 50% methanol was added to the resulting solution until the 25 ml mark of the contained was reached. The solution in amount of 1 ml was taken accurately and was transferred to a 10 ml-graduated container into which 50% methanol was added to the mark. The test solution was obtained after the solution was filtered by a 0.45 μm membrane.

The reference solution and the test solution were each taken out in an amount of 5 μL and were injected into the liquid chromatography column, and the peak areas of the echinacoside and the acteoside were measured. The contents were calculated by using the peak areas. The content of the echinacoside is 37.5% while the content of the acteoside is 6.7% by weight of the preparation.

Embodiment 6

6 kg of the end extract was dissolved in water with heating, which was in an amount of five times of the end extract. The extract solution was then applied into an adsorption column packed with pretreated macro-porous adsorption resin of the AB-8 type. The column was first eluted with water to yield a water eluate in the amount of eight times of the fleshy stems, and was than eluted with 60% ethanol to yield a first 60% ethanol eluate in the amount of eight times of the fleshy stems. The water eluate was subjected to another round of the adsorption-desorption operations by eluting the column with water in the amount of six times of the fleshy stems and with 60% ethanol in sequence to obtain a second ethanol eluate in the amount of seven times of the fleshy stems. The two 60% ethanol eluates were combined, concentrated, and dried to yield a preparation containing phenylethanoid glycosides and having a weight of 1203 g.

By using the HPLC method described in the Embodiment 5, the contents of echinacoside and acteoside are found to be respectively 48.6% by weight, and 11.8% by weight of the preparation.

Embodiment 7

6 kg of the end extract was dissolved in water with heating, which was in an amount of three times of the end extract. The extract solution was then applied into an adsorption column packed with macro-porous adsorption resin of the AB-8 type. The column was first eluted with water to yield a water eluate in the amount of eight times of the fleshy stems, and was than eluted with 95% ethanol to yield a first 95% ethanol eluate in the amount of eight times of the fleshy stems. The water eluate was subjected to another round of the adsorption-desorption operations by eluting the column with water in the amount of six times of the fleshy stems and with 95% ethanol in sequence to obtain a second ethanol eluate in the amount of eight times of the fleshy stems. The two 60% ethanol eluates were combined, concentrated, and dried to yield a preparation containing phenylethanoid glycosides and having a weight of 1260 g.

By using the HPLC method described in the Embodiment 5, the contents of echinacoside and acteoside are found to be respectively 41.3% by weight, and 7.4% by weight of the preparation.

Embodiment 8

6 kg of the end extract was dissolved in water with heating, which was in the same amount of the end extract. The extract solution was then applied into an adsorption column packed with macro-porous adsorption resin. The column was first eluted with water to yield a water eluate in the amount of four times of the fleshy stems, and was than eluted with 40% ethanol to yield a first 40% ethanol eluate in the amount of five times of the fleshy stems. The water eluate was subjected to another round of the adsorption-desorption operations by eluting the column with water in the amount of three times of the fleshy stems and with 40% ethanol in sequence to obtain a second ethanol eluate in the amount of four times of the fleshy stems. The two 40% ethanol eluates were combined, concentrated, and dried to yield a preparation containing phenylethanoid glycosides and having a weight of 1107 g.

By using the HPLC method described in the Embodiment 5, the contents of echinacoside and acteoside are found to be respectively 31.7% by weight, and 6.1 % by weight of the preparation.

PHARMACOLOGICAL EFFECTS:

Embodiment 9

The medicinal preparation of the present invention was used in a water maze experiment to study its effect on the learning memory of LACA mice. The Piracetam Tablets was used as a positive comparative drug for testing 6 days per week. The test lasted for 27 days. The test results are listed in Table 2.

Table 2 Effect on the leaning memory of mice by administering the preparation of the present invention orally

| Test | Comparison groups | d value (n=27) | | | | | |
|---|---|---|---|---|---|---|---|
| | | ≥0.5 | | ≥0.8 | | ≥0.5 sum | |
| | | day | % | day | % | day | % |
| First time | Control group - Group provided with 50 mg/kg of preparation containing phenylethanoid glycosides | 4 | 14.8 | 5 | 18.5 | 9 | 33.3 |
| | Control group - Group provided with 200 mg/kg of preparation containing phenylethanoid glycosides | 10 | 37.0 | 3 | 11.1 | 13 | 48.2 |
| Second time | Control group - Group provided with 400 mg/kg of reparation containing phenylethanoid glycosides | 0 | 0 | 27 | 100.0 | 27 | 100. 0 |
| | Control group - Positive comparative group provided with 400 mg/kg of Piracetam | 5 | 18.5 | 2 | 7.4 | 7 | 25.5 |
| | Piracetam group - Group provided with 400 mg/kg of reparation containing phenylethanoid glycosides | 7 | 25.9 | 16 | 59.3 | 23 | 85.2 |

According to the data listed in the Table 2, it is readily apparent that the preparation of the present invention has a meaningful effect on the learning memory of the mice, as exemplified by the difference in the response time between the control group and the doped groups, which were respectively doped with 50 mg/kg of the preparation containing phenylethanoid glycosides, 200 mg/kg of the preparation containing phenylethanoid glycosides, and 400 mg/kg of the preparation containing phenylethanoid glycosides. The period when the response time shows meaningful difference by d value analysis for the 50 mg/kg group is 9 days; for the 200 mg/kg group is 13 days; and for the 400 mg/kg group is 27 days. The period when the response time shows meaningful difference increases as the dosage increases. In addition, the period when the response time shows meaningful difference for the positive control group (Piracetam group) is 7 days, which is shorter than that of the doped group of 400 mg/kg, and thus Piracetam Tablets are less effective on the learning memory of mice compared to the preparation of the present invention.

Embodiment 10

The effect of the preparation of the present invention on the prevention of memory loss by the LACA mice was studied by the water maze experiment. The mice were orally administered with the drugs. One hour after such an oral administration, they were provided with 30% ethanol 0.1 ml/10 g BW. In 30 minutes, the water maze experiment was started. The results of the swimming performance of the mice were listed in Table 3.

Table 3 The effect of the preparation containing phenylethanoid glycoside one-week orally administered on the prevention of memory loss of LACA mail mice induced by ethanol

| Group | Dose (mg/kg) | Number of animal | Arrival at destination after being trained | | Arrival at destination after being given alcohol | | | |
|---|---|---|---|---|---|---|---|---|
| | | | Time (second) | Error number/ error number of animal | Time (second) | Error number/ error number of animal | Error rate, times/ number of animal | Group error rate, times/ number of animal |
| Preparation of the present invention | 50 | 12 | 7.46 ± 0.13 | 0/0 | 34.40 ± 21.71* | 47/8 | 5.88 | 3.92 |
| | 200 | 11 | 7.14 ± 0.18 | 0/0 | 16.99 ± 9.06## | 8/3△△ | 2.67 | 0.73 |
| | 400 | 10 | 7.91 ± 0.19 | 0/0 | 24.38 ± 27.84 | 46/7 | 6.57 | 4.60 |
| Piracetam control | 400 | 10 | 8.00 ± 0.46 | 0/0 | 24.08 ± 32.52 | 54/6 | 9.00 | 5.40 |
| | | 12 | 7.73 ± 0.75 | 0/0 | 37.78 ± 15.90 | 62/10 | 6.20 | 5.17 |

t study: comparison with the comparative group ## P<0.01, comparison with the 200mg/kg group *p<0.05 $X^2$ study: comparison with the comparative group △△ P<0.01

According to the data listed in Table 3, the medicinal preparation of the present invention is more effective in prevention of memory loss of the mice as compared with Piracetam. The arrival time of the group provided with the preparation of the present invention is shorter than that of the Piracetam group, after the mice were provided with ethanol. The difference between the preparation 200 mg/kg group and the control group is P<0.01. The difference between the preparation 50 mg/kg group and the preparation 200 mg/kg group is P<0.05. As a result, the does of the preparation is a factor. There is no significant difference among the preparation 400mg/kg group, the Piracetam group, and the control group, thereby indicating that the dose of 200 mg/kg is most effective in prevention of memory loss. On the basis of the "error" data listed in Table 3, the does of 200 mg/kg is most effective in prevention of memory loss.

Embodiment 11

A study was done on the effect of the preparation of the present invention on the memory gaining difficulty of the mice caused by Hyosine.

(1) drug and animal

The test drug and the comparative drug were identical with those of the Embodiment 9. The mice were grouped randomly into the normal comparative group, the model group, the Piracetam 600mg/kg group, and the preparation groups 400mg/kg, 200mg/kg, 100mg/kg. Each group contains 15 mice. The drugs were administered on the basis of 0.2 ml/10 g body weight. The jumping training was started on the completion of the 29th day administration. The drug was given one hour before training. With the exception of the normal comparative group, each group was provided with a belly cavity injection of Hyosine in amount of 1 mg/kg. The test was done on the 30th day, and the drug was given one hour before training. The results are listed on Table 4.

Table 4 The effect of phenylethanoid glycoside containing preparations on the mice memory gaining difficulty caused by hyosine ($\overline{X}$ ±SD)

| group | dose (mg/kg) | animal number | test latent (S) | frequency of test error (times/5min) |
|---|---|---|---|---|
| control group | | 14(1) | 194.1±101.5* | 1.36±1.45* |
| model group | | 15 | 67.1±78.4 | 3.13±2.47 |
| Piracetam group | 600 | 15 | 144.5±117.4* | 1.60±1.40* |

(continued)

| group | dose (mg/kg) | animal number | test latent (S) | frequency of test error (times/5min) |
|---|---|---|---|---|
| preparations of the present invention | 400 | 12(3) | 206.1±98.8** | 1.08±1.16** |
| | 200 | 12(3) | 183.2±115.2** | 1.42±1.38* |
| | 100 | 14(1) | 191.8±117.5** | 1.50±2.07 |
| comparison with the model group, *p<0.05, **p<0.01; the parenthesized numeral indicates the number of death caused by electric shock. | | | | |

According to the data listed in Table 4, the test latent periods of the groups are shortened as compared with the normal comparative group. However, the frequency of the test error increases. As compared with the model group, the Piracetam 600mg/kg group and the preparation groups 400 mg/kg, 200 mg/kg have a protracted latent period and a reduction in frequency of test error, thereby indicating a memory improvement. The preparation 100 mg/kg group has a protracted latent period, and an error frequency which is not different from that of the model group.

Reference example 1

A study was done on the effect of the preparation of the present invention on thrombosis of the vein bypass of the rats.

The male SD rats were studied as compared with the aspirin. The results are listed in Table 5.

Thrombosis inhibition rate (%) = (distilled water comparative group thrombosis weight - drug group thrombosis weight)/distilled water comparative group thrombosis weight × 100%.

Table 5 The effect of the preparation containing phenylethanoid glycoside administered orally on thrombosis of the vein bypass of rats

| Group | dose (mg/kg) | number of animal | thrombosis weight (mg, x±SD) | thrombosis inhibition rate (%) |
|---|---|---|---|---|
| distilled water | | 10 | 44.9±3.83 | |
| aspirin preparation | 100 | 10 | 29.2±4.00** | 34.97 |
| | 200 | 10 | 37.7±7.42* | 16.04 |
| | 100 | 10 | 36.1±5.16* | 19.60 |
| | 50 | 10 | 42.6±7.12 | 5.12 |
| comparison with the distilled water comparative group *p<0.05, **p<0.01. | | | | |

On the basis of the data listed in Table 5, the preparations 200 mg/kg and 100 mg/kg are capable of inhibiting the thrombosis of the vein bypass of the rats, as compared with the distilled water comparative group, with the inhibition rates being respectively 16.04%, 19.60%. However, the effects of the preparations are weaker than that of the aspirin 100 mg/kg which has an inhibition rate of 34.97%. The preparation 50 mg/kg has no effect on the thrombosis formation.

Reference Example 2

A study was done on the effect of the preparation of the present invention on aggregation of blood platelets of the rats.

The aggregation of blood platelets was caused by using adenosine disodium diphosphate (ADP). 1 mg/ml ADP solution was kept refrigerated before use. When the ADP solution was about to be used, it was diluted three times with the phosphoric acid buffer solution.

The male SD rats were randomly divided into five groups in accordance with the body weight. These five groups were the distilled water comparative group, the aspirin group, the 200 mg/kg preparation group, the 100 mg/kg preparation group, and the 50 mg/kg preparation group. The drugs were orally administered, with the administration volume being 0.5 mg/100 g. The rats of the distilled water comparative group were given distilled water in same quantity. The administration continued for 7 days. The rats were not fed for 12 hours prior to the final administration. One hour after the final administration the blood was drawn out from the main artery. The blood so draw out was prevented from aggregation by 3.8% sodium citrate (1:9). The blood sample was centrifuged at 1000 rpm for three minutes to facilitate the removing of the plasma rich in blood platelets (PRP). The remainder of the blood sample was further centrifuged at 300 rpm for 10 minutes to facilitate the separating of the plasma poor in blood platelets (PPP). 200 μL PRP was introduced into an opacity-comparison tube and the opacity thereof was adjusted to zero point with the PPP. The mixture was incubated for 5 minutes before adding thereinto 50 μL of the ADP solution for causing the aggregation of blood platelets. The aggregation degree was determined by using a SPA-4 multifunctional blood platelet aggregation meter. The inhibition rate of the blood platelet aggregation is calculated by the following formula.

$$\text{Inhibition rate (\%)} = \frac{\text{(control group maximum aggregation degree} - \text{experimental group maximum aggregation degree)}}{\text{(control group maximum aggregation degree)}} \times 100\%$$

The results of the test are listed in Table 6. It is readily apparent that the preparations (200 mg/kg, 100 mg/kg, 50 mg/kg) are capable of inhibiting the aggregation of blood platelets, and that the preparation (200 mg/kg) has the highest inhibition rate of 59.48%.

Table 6 The effect of preparation containing phenylethanoid glycosides orally administered on blood platelets aggregation of rats

| group | dose (mg/kg) | number of rats | maximum aggregation(%) (*±SD) | aggregation inhibition (%) |
|---|---|---|---|---|
| Distilled water group | | 10 | 54.82±7.88 | |
| Aspirin group | 100 | 11 | 32.73±11.14** | 40.30 |
| preparation | 200 | 10 | 22.21±6.23** | 59.48 |
| | 100 | 11 | 34.54±15.69* | 36.99 |
| | 50 | 10 | 31.65±12.81** | 42.26 |

Comparison with the distilled water group *P < 0.01, **P < 0.001

## Claims

1. A medicinal preparation containing phenylethanoid glycosides extracted from *Cistanche tubulosa (Schrenk.) Wight,* said preparation comprising 25-70% of echinacoside by weight of said preparation, and 5-40% of acteoside by weight of said preparation.

2. The preparation as defined in claim 1 which is extracted from fleshy stems of *Cistanche tubulosa (Schrenk.) Wight.*

3. The preparation as defined in claim 1 further comprising 2'-acetylacteoside; campneoside I; campneoside II; cistantubuloside A, $B_1$, $B_2$, $C_1$, $C_2$; crenatoside; decaffeoylacteoside; isoacteoside; rhodioloside; syringalide A; 3'-α-L-rhamnopyranoside, and tubuloside A, each being contained in an amount less than 5% by weight of said preparation.

4. A process for making a medicinal preparation containing phenylethanoid glycosides, said process comprising the following steps of:

a) extracting fleshy stems of *Cistanche tubulosa (Schrenk.) Wight* with a first polar solvent, wherein said first polar solvent is water, or a mixture of water and ethanol;

b) introducing the resulting extract from step a) into a column which is packed with hydrophobic macro-porous polymeric beads, thereby enabling phenylethanoid glycosides to be adsorbed on the polymeric beads;

c) eluting the column by use of a second polar solvent serving as a mobile phase, so that relatively less strongly adsorbed compounds are eluted from the column with most of phenylethanoid glycosides still being adsorbed on the polymeric beads wherein said second polar solvent is water;

d) eluting the column by use of a third polar solvent so as to obtain an eluate which contains phenylethanoid glycosides, wherein said third polar solvent is methanol, ethanol, a mixture of water and methanol, or a mixture of water and ethanol; and

e) removing the solvent that is contained in the eluate from step d), thereby resulting in production of a preparation comprising 25-70% of echinacoside and 5-40% of acteoside by weight of said preparation.

**5.** The process as defined in claim 4, wherein said extracting in step a) comprises mixing the fleshy stems of *Cistanche tubulosa (Schrenk.) Wight* with the first polar solvent, decocting the resulting mixture for 0.5-10 hours, and filtering the decocted mixture to obtain a liquid extract or concentrating the liquid in vacuo to obtain an extract in the concentrated form.

**6.** The process as defined in claim 4, wherein the polymeric beads in step b) are cross-linked polyaromatics.

**7.** The process as defined in claim 6, wherein the polymeric beads are formed of cross-linked polystyrene or cross-linked copolymer of styrene and divinyl benzene.

**8.** The process as defined in claim 4, wherein the third polar solvent is the mixture of water and ethanol.

**9.** A medicinal composition for use in the prevention of senile dementia, said medicinal composition comprising a therapeutically effective amount of the preparation as claimed in any one of claims 1 to 3 as an active ingredient, in admixture with a pharmaceutically acceptable carrier or diluent for the active ingredient.

**10.** Use of a medicinal preparation containing phenylethanoid glycosides extracted from *Cistanche tubulosa (Schrenk.) Wight* in the manufacture of a medicament for preventing and treating senile dementia disease in a patient, said preparation comprising 10-70% of echinacoside by weight of said preparation, and 1-40% of acteoside by weight of said preparation.

**11.** The use as defined in claim 10, wherein said preparation comprising 25-70% of echinacoside by weight of said preparation, and 5-40% of acteoside by weight of said preparation.

**12.** The use as defined in claim 10, wherein said preparation is extracted from fleshy stems of *Cistanche tubulosa (Schrenk.) Wight.*

**13.** The use as defined in claim 10, wherein said preparation further comprising 2'-acetylacteoside; campneoside I; campneoside II; cistantubuloside A, $B_1$, $B_2$, $C_1$, $C_2$; crenatoside; decaffeoylacteoside; isoacteoside; rhodioloside; syringalide A; 3'-$\alpha$-L-rhamnopyranoside, and tubuloside A, each being contained in an amount less than 5% by weight of said preparation.

**Patentansprüche**

**1.** Ein medizinisches Präparat, das Phenylethanoidglykoside enthält, die aus *Cistanche tubulosa* (Schrenk.) Wight extrahiert wurden, wobei das Präparat 25-70% Echinacosid bezogen auf das Gewicht des Präparats und 5-40% Acteosid, bezogen auf das Gewicht des Präparats umfasst.

**2.** Das Präparat, wie in Anspruch 1 definiert, das aus verdickten Stängeln der *Cistanche tubulosa* (Schrenk.) Wight extrahiert wird.

**3.** Das Präparat, wie in Anspruch 1 definiert, das weiterhin 2'-Acetylacteosid; Campneosid I; Campneosid II; Cistantubulosid A, $B_1$, $B_2$, $C_1$, $C_2$; Crenatosid; Dekaffeoylakteosid; Isoacteosid; Rhodiolosid; Syringalid A; 3'-a-L-Rhamnopyranosid, und Tubulosid A umfasst, die jeweils in einer Menge enthalten sind, die weniger als 5 Gew.-% des

Präparats beträgt.

**4.** Verfahren zur Herstellung eines medizinischen Präparats, das Phenylethanoidglykoside enthält, wobei das Verfahren die folgenden Schritte umfasst:

a) Extrahieren der verdickten Stängel der *Cistanche tubulosa* (Schrenk) Wight mit einem ersten polaren Lösungsmittel, wobei das erste polare Lösungsmittel Wasser oder eine Mischung von Wasser und Ethanol ist;
b) Einfügen des resultierenden Extrakts aus Schritt a) in eine Säule, die mit hydrophoben makro-porösen polymeren Kügelchen gepackt ist, wodurch die Phenylethanoidglykoside auf den polymeren Kügelchen absorbiert werden können;
c) Eluieren der Säule unter Verwendung eines zweiten polaren Lösungsmittels, das als mobile Phase dient, so dass relativ wenig stark absorbierte Verbindungen aus der Säule eluiert werden, wobei der Großteil der Phenylethanoidglykoside weiterhin auf den polymeren Kügelchen absorbiert sind, wobei das zweite polare Lösungsmittel Wasser ist;
d) Eluieren der Säule unter Verwendung eines dritten polaren Lösungsmittels um ein Eluat zu erhalten, das Phenylethanoidglykoside enthält, wobei das dritte polare Lösungsmittel Methanol, Ethanol, eine Mischung aus Wasser und Methanol, oder eine Mischung aus Wasser und Ethanol ist; und
e) Entfernen des Lösungsmittels, das im Eluat des Schritts d) enthalten ist, was in der Herstellung eines Präparats resultiert, das 25-70% Echinacosid und 5-40% Acteosid bezogen auf das Gewicht des Präparats umfasst.

**5.** Das Verfahren, wie in Anspruch 4 definiert, wobei das Extrahieren in Schritt a) das Mischen der verdickten Stängel der *Cistanche tubulosa* (Schrenk.) Wight mit dem ersten polaren Lösungsmittel, Auskochen der resultierenden Mischung für 0,5-10 Stunden und Filtrieren der ausgekochten Mischung umfasst, um einen flüssigen Extrakt zu erhalten, oder Konzentrieren der Flüssigkeit um einen Extrakt in konzentrierter Form zu erhalten.

**6.** Das Verfahren, wie in Anspruch 4 definiert, wobei die polymeren Kügelchen in Schritt b) vernetzte Polyaromaten sind.

**7.** Das Verfahren, wie in Anspruch 6 definiert, wobei die polymeren Kügelchen aus vernetztem Polystyrol oder aus vernetztem Copolymer aus Styrol und Divinylbenzol gebildet werden.

**8.** Das Verfahren, wie in Anspruch 4 definiert, wobei das dritte polare Lösungsmittel eine Mischung aus Wasser und Ethanol ist.

**9.** Eine medizinisches Zusammensetzung zur Verwendung bei der Vorbeugung von seniler Demenz, wobei die medizinisches Zusammensetzung eine therapeutisch wirksame Menge des Präparats, wie in einem der Ansprüche 1-4 beansprucht als Wirkstoff umfasst, in Beimischung mit einem pharmazeutisch verträglichen Träger oder Verdünnungsmittel für den Wirkstoff.

**10.** Verwendung eines medizinischen Präparats, das Phenylethanoidglykoside enthält, die aus *Cistanche tubulosa* (Schrenk.) Wight extrahiert wurden, zur Herstellung eines Medikaments zur Vorbeugung und Behandlung von seniler Demenz, wobei das Präparat 10-70% Echinacosid bezogen auf das Gewicht des Präparats und 1-40% Acteosid, bezogen auf das Gewicht des Präparats umfasst.

**11.** Die Verwendung, wie in Anspruch 10 definiert, wobei das Präparat 25-70% Echinacosid bezogen auf das Gewicht des Präparats und 5-40% Acteosid bezogen auf das Gewicht des Präparats umfasst.

**12.** Die Verwendung, wie in Anspruch 10 definiert, wobei das Präparat aus den verdickten Stängeln der *Cistanche tubulosa* (Schrenk.) Wight extrahiert wird.

**13.** Die Verwendung, wie in Anspruch 10 definiert, wobei das Präparat weiterhin 2'- Acetylacteosid; Campneosid I; Campneosid II; Cistantubulosid A, $B_1$, $B_2$, $C_1$, $C_2$; Crenatosid; Dekaffeoylakteosid; Isoacteosid; Rhodiolosid; Syringalid A; 3'-a-L-Rhamnopyranosid, und Tubulosid A umfasst, die jeweils in einer Menge enthalten sind, die weniger als 5 Gew.-% des Präparats beträgt.

## Revendications

**1.** Préparation médicamenteuse contenant des glycosides phényléthanoïdes extraits de *Cistanche tubulosa (Schrenk.)*

*Wight,* ladite préparation comprenant 25 à 70 % d'échinacoside en poids de ladite préparation, et 5 à 40 % d'actéoside en poids de ladite préparation.

**2.** Préparation selon la revendication 1 qui est extraite des tiges charnues de *Cistanche tubulosa (Schrenk.) Wight.*

**3.** Préparation selon la revendication 1 comprenant en outre du 2'-acétylactéoside ; du campnéoside I ; du campnéoside II ; du cistantubuloside A, $B_1$, $B_2$, $C_1$, $C_2$ ; du crénatoside ; du décaféoylactéoside ; de l'isoactéoside ; du rhodioloside ; du syringalide A ; du 3'-$\alpha$-L-rhamnopyranoside, et du tubuloside A, chacun étant contenu en une quantité inférieure à 5 % en poids de ladite préparation.

**4.** Procédé de fabrication d'une préparation médicamenteuse contenant des glycosides phényléthanoïdes, ledit procédé comprenant les étapes suivantes :

a) extraction de tiges charnues de *Cistanche tubulosa (Schrenk.) Wight* avec un premier solvant polaire, ledit premier solvant polaire étant de l'eau, ou un mélange d'eau et d'éthanol ;
b) introduction de l'extrait résultant de l'étape a) dans une colonne qui est garnie de billes polymères macroporeuses hydrophobes, permettant ainsi aux glycosides phényléthanoïdes d'être adsorbés sur les billes polymères ;
c) élution de la colonne au moyen d'un second solvant polaire servant de phase mobile, de sorte que les composés relativement moins fortement adsorbés sont élués de la colonne tandis que la majorité des glycosides phényléthanoïdes restent encore adsorbés sur les billes polymères, ledit second solvant polaire étant de l'eau ;
d) élution de la colonne au moyen d'un troisième solvant polaire de façon à obtenir un éluat qui contient des glycosides phényléthanoïdes, ledit troisième solvant polaire étant du méthanol, de l'éthanol, un mélange d'eau et de méthanol, ou un mélange d'eau et d'éthanol ; et
e) élimination du solvant qui est contenu dans l'éluat de l'étape d), aboutissant ainsi à la production d'une préparation comprenant 25 à 70 % d'échinacoside et 5 à 40 % d'actéoside en poids de ladite préparation.

**5.** Procédé selon la revendication 4, dans lequel ladite extraction dans l'étape a) comprend le mélange des tiges charnues de *Cistanche tubulosa (Schrenk.) Wight* avec le premier solvant polaire, la décoction du mélange résultant pendant 0,5 à 10 heures, et la filtration du mélange ayant subi la décoction pour obtenir un extrait liquide ou la concentration du liquide sous vide pour obtenir un extrait sous forme concentrée.

**6.** Procédé selon la revendication 4, dans lequel les billes polymères dans l'étape b) sont des polyaromatiques réticulés.

**7.** Procédé selon la revendication 6, dans lequel les billes polymères sont formées de polystyrène réticulé ou d'un copolymère réticulé de styrène et de divinylbenzène.

**8.** Procédé selon la revendication 4, dans lequel le troisième solvant polaire est le mélange d'eau et d'éthanol.

**9.** Composition médicamenteuse destinée à être utilisée dans la prévention de la démence sénile, ladite composition médicamenteuse comprenant une quantité thérapeutiquement efficace de la préparation selon l'une quelconque des revendications 1 à 3 en tant que principe actif, en mélange avec un véhicule ou diluant pharmaceutiquernent acceptable pour le principe actif.

**10.** Utilisation d'une préparation médicamenteuse contenant des glycosides phényléthanoïdes extraits de *Cistanche tubulosa (Schrenk.) Wight* dans la fabrication d'un médicament destiné à prévenir et à traiter la démence sénile chez un patient, ladite préparation comprenant 10 à 70 % d'échinacoside en poids de ladite préparation, et 1 à 40 % d'actéoside en poids de ladite préparation.

**11.** Utilisation selon la revendication 10, dans laquelle ladite préparation comprend 25 à 70 % d'échinacoside en poids de ladite préparation, et 5 à 40 % d'actéoside en poids de ladite préparation.

**12.** Utilisation selon la revendication 10, dans laquelle ladite préparation est extraite des tiges charnues de *Cistanche tubulosa (Schrenk.) Wight.*

**13.** Utilisation selon la revendication 10, dans laquelle ladite préparation comprend en outre du 2'-acétylactéoside ; du campnéoside I ; du campnéoside II ; du cistantubuloside A, $B_1$, $B_2$, $C_1$, $C_2$ ; du crénatoside ; du décaféoylactéoside ; de l'isoactéoside ; du rhodioloside ; du syringalide A ; du 3'-$\alpha$-L-rhamnopyranoside, et du tubuloside A, chacun étant

contenu en une quantité inférieure à 5 % en poids de ladite préparation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SATO T. et al.** *Yakugaku Zasshi,* 1985, vol. 105 (12), 1131
- **JIMENEZ C. et al.** *Nat Prod Rep,* 1994, vol. 11 (6), 591
- **COMETA F. et al.** *Fitoterapia,* 1993, vol. 64 (3), 195